# EUROPEAN PATENT APPLICATION

(11) **EP 3 763 700 A1**
(43) Date of publication of application: **13.01.2021**
(21) Application number: 19185472.8
(22) Date of filing: 10.07.2019
(51) Int. Cl.: C07C 303/44, C07C 309/04, C07C 309/06, C07C 309/20, C07C 309/25, C07C 309/35, C07C 309/39

(54) **IMPROVED PROCESS FOR THE PURIFICATION OF SULFONIC ACIDS**

(71) Applicant: Grillo-Werke Aktiengesellschaft, 47169 Duisburg (DE)
(72) Inventor: OTT, Timo, 47169 Duisburg (DE); BIERTÜMPEL, Ingo, 47169 Duisburg (DE)
(74) Representative: dompatent von Kreisler Selting Werner - Partnerschaft von Patent- und Rechtsanwälten mbB

(57) **Abstract**

The present application discloses a process to provide purified sulfonic acid R-SO₃H, wherein R represents a moiety comprising 1 or more C-atoms except for CH₃, the process comprising the following steps:
a) providing a liquid mixture comprising sulfonic acid R-SO₃H,
b) adjusting the mixture such that the concentration of sulfonic acid is at least 30 % by weight, based on the total amount of the mixture,
c) distillation or rectification of the mixture.

## Description

The present invention refers to an improved process for the purification of sulfonic acids R-SO₃H, in which the concentration of sulfonic acid in a mixture is set such that purification by distillation is improved.

Sulfonic acids are any of a class of organic acids containing sulfur and having the general formula R-SO₃H, in which R is an organic combining group. The sulfonic acids are among the most important of the organosulfur compounds; the free acids are widely used as catalysts in organic syntheses, while the salts and other derivatives form the basis of the manufacture of detergents, watersoluble dyes and catalysts, sulfonamide pharmaceuticals, and ion-exchange resins. Aromatic sulfonic acids are particularly useful as intermediates or starting materials in synthesis, for example, in the preparation of phenols. Sulfonic acid groups can greatly enhance the water solubility of compounds, as seen with the sulfonic acid derivative of triphenyl phosphine (TPPTS), P(C₆H₄-m-SO₃Na)₃. Metal complexes of this compound are used as homogeneous catalysts for the syntheses of organic compounds in two-phase systems (e.g., in a mixture of water and an organic solvent) in industry and in the laboratory. Aromatic sulfonic acids are obtained generally by treating aromatic compounds with concentrated sulfuric acid and added sulfur trioxide ("oleum"), the process being called sulfonation.

The resulting reaction mixture of sulfonation processes usually comprises the sulfonic acid as product as well as SO₃ as non-reacted educt together with eventually other non-reacted educts and side products. Usually, it is intended to remove this excess of SO₃. This is commonly performed by addition of water resulting in the formation of H₂SO₄. To obtain the pure sulfonic acid without H₂SO₄ or other impurities and side products, a purification step is performed. Usually, the mixture is distilled. But the conditions of distillation are highly dependent on the concentration of sulfuric acid in the mixture. Surprisingly it was found that it is possible to provide sulfonic acid in high quality with low level impurities by increasing the amount of sulfonic acid in a liquid mixture prior to distillation or rectification.

In a first embodiment, the problem of the present application is thus solved by a process to provide purified sulfonic acid R-SO₃H, wherein R represents a moiety comprising 1 or more C-atoms except for CH₃, the process comprising the following steps:
a) providing a liquid mixture comprising sulfonic acid R-SO₃H,
b) adjusting the mixture such that the concentration of sulfonic acid is at least 30 % by weight, based on the total amount of the mixture,
c) distillation or rectification of the mixture.

In a preferred embodiment, the process further comprises the step
d) purifying the sulfonic acid obtained from step c) by adsorption methods.

The inventors of the present application surprisingly found that it helps to improve the quality of the sulfonic acid if the concentration of sulfonic acid in the reaction mixture which is distilled is above 30 % by weight, based on the total weight of the mixture. The higher the concentration of sulfonic acid in the mixture, the lower is the energy need for the distillation, as the distillation can be performed at lower temperatures compared to temperatures needed for lower concentrations of sulfonic acid.

The sulfonic acid according to the present invention has the general formula R-SO₃H, wherein R- is a moiety with 1 or more C-atoms, with the exclusion of CH₃.

R may be a linear or branched, substituted or unsubstituted alkyl or aryl group. R may also be a cycloalkyl or heteroaryl group. R may also be a polymer, preferably a polyolefine, such as polyethylene or polypropylene. Other suitable polymers are also polystyrene, polyester, copolymers of polystyrene, such as styrene-butadiene copolymers, high impact polystyrene (PS-I), styrene-butadiene-blockcopolymers, or styrene-butadiene-rubber.

As used throughout herein, unless stated otherwise, "alkyl" means carbon chains which may be linear (straight chain) or branched. Examples of, for example, C₂ₜₒ₄alkyl groups include ethyl, n-propyl, isopropyl, n-, iso-, sec- and tert-butyl. As used throughout herein, unless stated otherwise, "cycloalkyl" means rings of which the atoms forming the ring itself are exclusively carbon atoms. The term "halogen" as used throughout herein means, unless otherwise stated, F, CI, Br or I. As used herein, the term "halogen" preferably means F, CI or Br, especially preferred F. As used throughout herein, the term "heteroaryl" rings means 5- or 6-membered unsubstituted or substituted N-containing heteroaryl rings containing up to 2 additional heteroatoms independently selected from N, O and S. Examples of such heteroaryl rings are pyrrolyl, pyrazolyl, imidazolyl, oxazolyl, isoxazolyl, thiazolyl, isothiazolyl, triazolyl, oxadiazolyl, thiadiazolyl, pyridinyl, pyridazinyl, pyrimidinyl, pyrazinyl, triazinyl, indolyl. The term "aryl" as used throughout herein may refer to unsubstituted aryl and/or substituted aryl. Compounds described throughout herein may contain one or more asymmetric centers and may thus give rise to diastereomers and optical isomers. The present invention includes all such possible diastereomers as well as their racemic mixtures, their substantially pure resolved enantiomers, and all possible geometric isomers. Further, mixtures of stereoisomers as well as isolated specific stereoisomers are also included.

In an exemplary aspect of the invention, an "aryl" group is preferably an unsaturated aromatic carbocyclic group of from 6 to 14 carbon atoms having a single ring (e.g., phenyl) or multiple condensed rings (e.g., naphthyl or anthryl). Particular aryls include phenyl, biphenyl, naphthyl and the like.

In an exemplary aspect of the invention, a "heteroaryl" or "heteroaromatic" ring/group is preferably an aryl ring system having one to four heteroatoms (e.g., O, S, N, or combinations thereof) as ring atoms in a heteroaromatic ring system, wherein the remainder of the atoms are carbon atoms. The heteroaryl moiety preferably may consist of a single or fused ring system. A typical single heteroaryl ring in this sense is a 5- to 6-membered ring containing one to four, preferably one to three or one to two heteroatoms selected from oxygen, sulfur and nitrogen and a typical fused heteroaryl ring system is a 9- to 10-membered ring system containing one to four heteroatoms selected from oxygen, sulfur and nitrogen. Examples of 5-membered (single ring) heteroaryl include pyrrolyl, furanyl, thienyl, imidazolyl, pyrazolyl, oxazolyl, isoxazolyl, thiazolyl, isothiazolyl, triazolyl, furazanyl, oxadiazolyl, thiadiazolyl, dithiazolyl, and tetrazolyl. Examples of 6-membered (single ring) heteroaryl include pyridyl, pyrazinyl, pyrimidyl, pyridazinyl, triazinyl, and tetrazinyl. Examples of fused heteroaryl ring systems include indolyl, oxindolyl, indazolyl, coumarinyl, 1H-indolyl, 1H-indazolyl, benzo[d]thiazolyl, benzofuranyl, purinyl, benzimidazolyl, quinolinyl, isoquinolinyl, benzothiophenyl, benzoxazolyl, 1,2-diazanaphthyl, 1,3-diazanaphthyl, 1,4-diazanaphthyl, 1,5-diazanaphthyl, 1,6-diazanaphthyl, 1,7-diazanaphthyl, 1,8-diazanaphthyl, 2,3-diazanaphthyl, 2,6-diazanaphthyl, and 2,7-diazanaphthyl. The heteroaryl group may preferably be attached to the chemical entity or moiety of the compounds of the invention to which they are bonded by any one of the atoms within the aromatic ring system (e.g., imidazol-1-yl, imidazol-2-yl, imidazol-4-yl, imidazol-5-yl, pyrid-2-yl, pyrid-3-yl, pyrid-4-yl, pyrid-5-yl, or pyrid-6-yl). In one embodiment, the heteroaryl group is a 5- to 10-membered heteroaryl group.

Examples of 5-membered (single ring) heteroaryl include pyrrolyl, furanyl, thienyl, imidazolyl, pyrazolyl, oxazolyl, isoxazolyl, thiazolyl, isothiazolyl, triazolyl, furazanyl, thiadiazolyl, and tetrazolyl.

Examples of 6-membered (single ring) heteroaryl include pyridyl, pyrazinyl, pyrimidyl, pyridazinyl, triazinyl. Examples of bicyclic fused ring heteroaryl include indolyl, benzofuranyl, indazolyl, oxindolyl, benzimidazolyl, benzothiophenyl, benzoxazolyl, benzo[d]thiazolyl, quinolinyl, isoquinolinyl, coumarinyl, purinyl, 1,2-diazanaphthyl, 1,3-diazanaphthyl, 1,4-diazanaphthyl, and 1,5-diazanaphthyl.

In the following, preferred embodiments for R- are provided.

If R is alkyl, it is preferably ethyl or propyl or butyl, or any other linear or branched alkyl group with 5 to 20 C-atoms, for example hexyl, heptyl, octyl, nonyl, decyl, dodecyl, tetradecyl, hexadecyl, or octadecyl.

If R is a substituted alkyl, it may by any alky with one or more C-Atoms, in which 1 or more H-atoms replaced by a functional group. If the alkyl comprises 2, 3, 4 or more C-atoms, one or more H-atoms may be replaced at one, two, three or all of the C-atoms. Thus, only some of the C-atoms of the alkyl group may be functionalized and at each C one, or more H-atoms may be replaced by the functional group. The functional group is selected from a group consisting of C₂ₜₒ₄alkyl, halogen, hydroxyl, -OCH₃, -OC₂ₜₒ₄alkyl, amino, ethynyl, -OCF₃, and -CF₃, -(CH₂)ₙN(CH₃)₂ where n is an integer from 1 to 3. It is also with the scope of the invention, that R may comprise functional groups replacing one or more of a CH₃ group or CH₂ group of the alkyl residue or one or more C-atoms in substituted alkyl groups. R may therefore comprise a carbonyl group, acyl halide group, carbonate group, carboxylate group, carboalkoxy group, alkoxy group, carboxamide group, amine group, imine group, imide group, azo group, oxime group, sulphide group, disulfide group, sulfinyl group, sulfonyl group, sulfo group, thiocyanate group, carbonothioyl group, thioester group, phosphino group, phosphono groupphosphate group, borono group, borinate group, borino group or boronate group.

If R is aryl they are preferably substituted or unsubstituted phenyl, and when they are substituted phenyl they are preferably phenyl substituted with one or more substituents independently selected from the group consisting of C₂ₜₒ₄alkyl, halogen, hydroxyl, -OCH₃, -OC₂ₜₒ₄alkyl, amino, ethynyl, -OCF₃, and -CF₃,-(CH₂)ₙN(CH₃)₂ where n is an integer from 1 to 3. Halogen is preferably F, CI, Br, or I, especially F, CI, or Br.

Preferably, R-SO₃H is selected from the group consisting of ethyl sulfonic acid, propane sulfonic acid, butane sulfonic acid, dodecane sulfonic acid, anthracene sulfonic acid, naphthalene sulfonic acid, perylene sulfonic acid, arylphosphane sulfonic acid, aryl mine sulfonic acid, mono-fluoromethyl sulfonic acid, di-fluoromethyl sulfonic acid, tri-fluoromethyl sulfonic acid, mono-iodomethyl sulfonic acid, mono-bromomethyl sulfonic acid, or the corresponding ethyl or propyl sulfonic acid, halogenarylmethyl sulfonic acids (for example X-C₆H₆-CH₂-SO₃H, with X=F, CI, Br, I), polyolefin sulfonic acid, polyester sulfonic acid and/or polyester sulfonic acid.

If R is a polymer, it is within the scope that not only one monomer of the polymer comprises the SO₃H functionality. Di- or poly-sulfonic acids are within the scope of the present invention as well. It is further within the scope of the invention that the SO₃H functionality is not only in one position but in several positions for alky or aryl residues for R, wherein the SO₃H can bind to different C-atoms or two or three SO₃H groups can bind to 1 C-atom, for example.

Preferably, the concentration of sulfonic acid R-SO₃H in the mixture is therefore in a range from 35 % by weight to 95 % by weight or to 90 % by weight, especially from 40 % by weight to 85 % by weight, preferred from 45 % by weight to 80 % by weight, advantageously from 50 % by weight to 75 % by weight, especially preferred from 55 % by weight to 70 % by weight or from 60 % by weight to 65 % by weight. The amounts of weight always refer to the total weight of the mixture, which is 100 % by weight.

Thus, in cases where the amount of sulfonic acid R-SO₃H in the reaction mixture is below the above-mentioned value, it is within the scope of the invention to increase the concentration of sulfonic acid in the reaction mixture, meaning at the same time that the concentration of other compounds in the mixture is reduced. Thus, the process of the present invention not only describes an increase of concentration of sulfonic acid R-SO₃H but also decrease of certain types of impurities, especially sulfate containing impurities.

A mixture within the scope of the present application can be a mixture resulting from a process of producing sulfonic acid R-SO₃H. Different ways of producing sulfonic acid R-SO₃H are known. A reaction mixture within the meaning of the present application is especially a mixture resulting from a method of producing sulfonic acid R-SO₃H by a sulfonation process.

A mixture within the meaning of the present application could also be a mixture which is obtained after sulfonic acid R-SO₃H was used in different technical fields. For example, in processes where sulfonic acid R-SO₃H is used as solvent or as catalyst for example, it is of interest to recycle the sulfonic acid R-SO₃H. This recycling can be performed by distillation for example. To receive also here high quality of sulfonic acid R-SO₃H after distillation, it is also advisable to increase the amount of sulfonic acid R-SO₃H in the mixture prior to distillation. Therefore, the mixture within the meaning of the present application is not only a reaction mixture obtained from a process to produce sulfonic acid R-SO₃H, but any mixture comprising sulfonic acid R-SO₃H. Liquid within the meaning of the present invention means liquid under ambient conditions (20 °C and 1 bar pressure)

After distillation of the mixture, sulfonic acid R-SO₃H is obtained as product. The sulfonic acid R-SO₃H might still comprise impurities, especially sulphate impurities. Reducing the concentration of these impurities can be performed by adsorption processes according to the present invention.

Sulfonic acid anhydrides are for example often formed during distillation. The higher the temperature during distillation, the higher the concentration of the anhydride formed during distillation. The process of the present invention now enables to perform the distillation at lower temperatures due to the higher concentration of sulfonic acid R-SO₃H in the mixture.

To obtain a pure sulfonic acid R-SO₃H, it is within the scope of the present invention to either increase the concentration of sulfonic acid R-SO₃H and decrease the concentration of impurities prior to distillation or to purify the sulfonic acid R-SO₃H obtained after the distillation. It is also within the scope to perform both, namely first increasing the concentration of sulfonic acid R-SO₃H in the mixture and further purify the sulfonic acid R-SO₃H obtained after distillation.

The increase of the sulfonic acid R-SO₃H or decrease of sulphate impurities in step b) of the present invention is preferably performed by melt crystallization and/or sorption.

If melt crystallization is used to increase the concentration of sulfonic acid R-SO₃H, it is essential that the reaction mixture comprises either some amounts of water or excess of sulfur trioxide should be present. In case where water and sulfonic acid R-SO₃H are present, it is assumed that the monohydrate of sulfonic acid R-SO₃H may be formed, which can be crystallized at temperatures around -5 °C to -50 °C, especially at -10 °C to -25 °C. The molar ratio between sulfonic acid R-SO₃H and water in the reaction mixture should thus be in the range between 1:5 to 1:1, preferably about 1:2 to 1:4 or 1:3 (always meaning the molar ratio sulfonic acid R-SO₃H : water).

Alternatively, if the crystallization cannot be performed as the addition of water shall be avoided, it is also possible to add some excess of sulfur trioxide, enabling again the formation of crystals at a temperature between -5 °C to - 15 °C. here, the molar ratio between sulfonic acid R-SO₃H and sulfur trioxide is preferably within the range of from 1:3 to 1:1 or 1:2.

Surprisingly, it was found that an increase of sulfonic acid R-SO₃H of about 2-20% by weight was performed. The amount means here absolute values. For example, if the original reaction mixture comprises 30% by weight of sulfonic acid R-SO₃H, it was possible to increase the concentration of sulfonic acid R-SO₃H to be close to 50% by weight. This would mean an increase of about 20% by weight in absolute amount.

The increase of the sulfonic acid R-SO₃H was observed in case where the crystals are separated from the liquid after the crystallization. The melt of the crystals can then be used for the distillation step. At the same time, this also means that in the liquid phase, the amount of sulfonic acid R-SO₃H was decreased.

The increase of sulfonic acid or decrease of especially sulphate impurities may also be performed by adsorption processes, especially by physisorption and/or chemisorption. The adsorption is preferably performed with a membrane and/or a porous structure. Suitable membranes are ceramic nanofiltration membranes, where the cut off depends on the size of the sulfonic acid and the impurities to be removed. Preferably, the mixture is water-free for nanofiltration applications, as water-free systems enable a higher increase of the concentration of sulfonic acid in the mixture by ceramic membranes. The increase of concentration of sulfonic aid was about 1% by weight to 5% by weight in absolute values (as defined above).

A physisorption processes can also be used to increase the concnetration of sulfonic acid. Respective porous structures can be provided as solid additives. Their porous size is to be adapted to the size of the impurities which should be reduced. At the same time, the size of sulfonic acid has to be kept in mind so that the sulfonic acid R-SO₃H is not adsorbed by the porous structure. Suitable porous structures are molecular sieves, silicon oxide, aluminium oxide or zeolites.

If by the above-mentioned methods, the amount of sulfonic acid R-SO₃H in the reaction mixture is increased to an amount, which is suitable to perform the distillation, this is performed with at least one distillation column. This distillation column can be adapted in its stages. Thus, for example, a reaction mixture comprising sulfonic acid R-SO₃H, sulfuric acid, water and SO₃ is provided as mixture. In a first step, the amount of sulfonic acid R-SO₃H in the reaction mixture is increased by any of the above described methods. This reaction mixture is then provided into a distillation means. It is possible to use the product obtained from the distillation means again to provide it in the reaction mixture obtained after increasing the amount of sulfonic acid R-SO₃H to further increase the concentration in the mixture which is provided into the distillation means.

It is further preferred, that the reaction mixture is provided directly into the distillation tower. In a preferred embodiment, there is a heating element provided prior to the inlet, where the reaction mixture is added into the distillation tower. This heating element enables a constant temperature in the distillation column so that a good separation of sulfonic acid R-SO₃H from other compounds of the reaction mixture can be obtained.

In the bottom of the distillation means, the still, there is a solution comprising sulfonic acid R-SO₃H in lower concnentration. It is of course possible, to circulate this liquid and also bring it into contact with a heating element to have a temperature as constant as possible.

The distillation column is heated and isolated, to ensure that the temperature inside the column is as constant as possible. The material of the distillation means can be any material which is resistant against the acidity of sulfonic acid R-SO₃H and any other acid which may be present in the reaction mixture. Preferably, the material is selected from glass, Teflon, silicon carbide, ceramic materials, porcelain, especially glazed porcelain, emaille, stainless steel, especially with an emaille coating. The material has to be selected such that there is no direct contact between a metal and the reaction mixture, as sulfonic acid R-SO₃H and may be other assets can react with different kinds of metals or metals can catalyse any unwanted side reaction.

When heating the distillation column and the still, it must be controlled that the temperature is always homogenously distributed. It should be avoided that at certain positions high temperatures are rising. This can be performed by mixing, e.g. by stirring, or circulating the mixture in the still.

The distillation may be performed within one distillation column. It is also within the scope of the invention that 2, 3 or more distillation columns are used. The length of the distillation column can be adjusted such that in the still there is only sulfuric acid present. This enables an extremely high temperature of the still but has the disadvantage of lot of space is needed for such a process.

Thus, there are several parameters in the distillation which can be adapted to obtain a sulfonic acid R-SO₃H which as pure as possible. At the same time the formation of side products can be controlled.

Nevertheless, it is still possible that during distillation side products are formed. Especially for the removal of such side products but also of other impurities which could not be removed by distillation, a purification step after distillation may be performed. This additional purification step is preferably performed as physorption and/or chemisorption process. A porous structure, especially activated carbon, a molecular sieve, silicon oxide, aluminium oxide and/or zeolite may be used as physisorption of sulphate impurities. Again, as already discussed for the increasing step, the size of the pores of the porous structure has to be adapted such that the impurity is adsorbed at the porous structure, but the sulfonic acid R-SO₃H is not.

Preferably, the purification after the distillation is performed via chemisorption. Here a chemical compound can be used, having a functionalization forming a nucleophilic group. This nucleophilic group is able to form anions which then react with side products, especially with side products comprising highly reactive R- residues, namely alkyl or aryl residues.

Suitable polymers are for example polyethylene, polypropylene and/or polystyrene. These polymers may have for example an amino functionalization or a hydroxy functionalization. This means of course, that there is not necessarily only one functional group present in the polymer but several may be present. It is also possible that a polymer comprises amino and hydroxy-functional groups.

## Claims

1. Process to provide purified sulfonic acid R-SO₃H, wherein R represents a moiety comprising 1 or more C-atoms except for CH₃, the process comprising the following steps:
a) providing a liquid mixture comprising sulfonic acid R-SO₃H,
b) adjusting the mixture such that the concentration of sulfonic acid is at least 30 % by weight, based on the total amount of the mixture,
c) distillation or rectification of the mixture.

2. Process according to claim 1, wherein the process further comprises the step
d) purifying the sulfonic acid R-SO₃H obtained from step c) by adsorption methods.

3. Process according to claim 1 or 2, wherein the increase in step b) is performed by melt crystallisation and/or adsorption.

4. Process according to claim 3, wherein the adsorption is a physisorption and/or chemisorption.

5. Process according to claim 3, wherein the adsorption is performed with a membrane and/or a porous structure, especially a molecular sieve.

6. Process according to any of claims 1 to 5, wherein the mixture comprises methane sulfonic acid, SO₃, methane and sulfuric acid.

7. Process according to any of claims 1 to 6, wherein the purification in step d) is performed as physisorption and/or chemisorption.

8. Process according to claim 7, wherein the physisorption is performed at a porous structure, especially activated carbon, molecular sieve, silicon oxide, aluminum oxide and/or zeolite.

9. Process according to claim 7, wherein the chemisorption is performed with a functionalized polymer, especially with a polymer having at least one amino and/or hydroxy-functional group.

10. Process according to claims 9, wherein the polymer is polyethylene, polypropylene and/or polystyrene.

11. Process according to any of claims 1 to 10, wherein R-SO₃H is selected from the group consisting of ethyl sulfonic acid, propane sulfonic acid, butane sulfonic acid, dodecane sulfonic acid, anthracene sulfonic acid, naphthalene sulfonic acid, perylene sulfonic acid, arylphosphane sulfonic acid, aryl amine sulfonic acid, mono-fluoromethyl sulfonic acid, di-fluoromethyl sulfonic acid, tri-fluoromethyl sulfonic acid, mono-iodomethyl sulfonic acid, mono-bromomethyl sulfonic acid, or the corresponding ethyl or propyl sulfonic acid, halogenarylmethyl sulfonic acids (for example X-C₆H₆-CH₂-SO₃H, with X=F, CI, Br, I), polyolefin sulfonic acid, polyester sulfonic acid and/or polyester sulfonic acid.

12. Process according to any of claims 1 to 11, wherein R-SO₃H is selected from ethyl sulfonic acid, dodecane sulfonic acid, mono-fluoromethyl sulfonic acid, tri-fluoromethyl sulfonic acid, and polyolefin sulfonic acid.
